# EUROPEAN PATENT SPECIFICATION

(11) **EP 3 167 826 B1**
(45) Date of publication and mention of the grant of the patent: **26.09.2018**
(21) Application number: 16198337.4
(22) Date of filing: 11.11.2016
(51) Int. Cl.: A61B 17/34, A61B 17/32

(54) **BALLOON DISSECTION KIT WITH MULTIPLE BALLOONS**
BALLONDISSEKTIONSKIT MIT MEHREREN BALLONS
KIT DE DISSECTION À BALLONNET AVEC BALLONNETS MULTIPLES

(30) Priority: 12.11.2015 US 201562254391 P; 02.11.2016 US 201615341021
(43) Date of publication of application: 17.05.2017
(73) Proprietor: Covidien LP, Mansfield, MA 02048 (US)
(72) Inventor: HARTOUMBEKIS, Elias, New Haven, CT 06512 (US); BREINDEL, Jay, Kensington, CT 06037 (US)
(74) Representative: Soames, Candida Jane

(56) References cited:
- DE-A1- 10 254 503
- GB-A- 2 397 023
- US-A- 5 359 995
- US-A- 5 707 382
- US-A1- 2013 023 733
- US-A1- 2013 110 151
- US-B1- 7 938 842

## Description

### CROSS-REFERENCE TO RELATED APPLICATIONS

This application claims the benefit of and priority to U.S. Provisional Patent Application No. 62/254,391 filed November 12, 2015.

### TECHNICAL FIELD

The present disclosure relates to dissection devices for forming an anatomical space within a body, and, in particular, to balloon dissection devices, with multiple detachable balloons permitting selection of the optimal balloon for use with a patient.

### BACKGROUND

During certain surgical procedures, it is necessary to dissect tissue layers to form an anatomical space for accessing a surgical site, and within which surgical instruments may be manipulated. For example, in hernia repair surgery, it is necessary to form an anatomical operative cavity within the extra peritoneal space in order to dissect fascia tissue layers from the peritoneum and access the hernia site. Various balloon dissectors are known for performing the tissue dissection procedure used in hernia repair surgery. Depending on the patient and procedure being performed, different shapes and sizes of balloons are used in performing tissue dissection procedures.

While the currently known tissue dissection devices are useful, it would be beneficial to have a kit possessing multiple detachable balloons to optimize use with individual patients in surgical procedures requiring dissection of tissue layers.

GB 2397023 A discloses a tissue dissecting hernia repair balloon kit including a balloon for attachment to a laparoscope and a thread tie for securing the balloon thereon.

US 7938842 B1 discloses a dissection cannula with a detachable dilating element coupled to the outer surface of the cannula for facilitating atraumatic expansion. The interchanging dilating element can be connected with a threaded connection or with a mating lock.

### SUMMARY

The invention is described in the independent claims and preferred embodiments are listed in the dependent claims.

The present disclosure is directed to a dissection and access kit including a balloon dissector assembly having a balloon dissector shaft and a plurality of detachable balloons. Each balloon is releasably attachable to the balloon dissector shaft prior to use and then disconnected following use in order to be disposed. By providing balloons that are releasably secured to the balloon dissector shaft, a clinician is able to select a balloon best suited to a particular surgical procedure.

In one aspect of the disclosure, a kit for a dissection and access system includes a cannula assembly having a cannula housing and an access cannula, an obturator insertable into the access cannula, and a dissector assembly insertable into the access cannula. The dissector assembly has an elongate shaft and a distal tip. At least one dissection balloon includes an inflatable portion and a connector for releasable attachment to the distal tip.

In embodiments, at least one spacer is releasably attached to the elongate shaft for limiting a depth of insertion of the elongate shaft of the dissector assembly into a body of a patient.

In some embodiments, a seal is provided for placement between the distal tip of the elongate shaft and the connector.

In certain embodiments, the distal tip of the elongate shaft and the connector possess threads permitting the distal tip and connector to be threadably attached.

In embodiments, the distal tip of the elongate shaft is releasably attached to the connector by a bayonet configuration.

In some embodiments, the distal tip possesses an open end with slots on an inner surface of the elongate shaft. The slots extend proximally along the inner wall of the elongate shaft to form recesses thereon. The connector possesses a proximal end configured to fit within the open end of elongate shaft. An outer surface of the proximal end possesses tabs that extend from the outer surface of the connector and are configured to be slidably received within the slots of the open end of the elongate shaft.

In certain embodiments, the distal tip of the elongate shaft is releasably attached to the connector by a locking collar.

In embodiments, the at least one dissection balloon includes a plurality of round balloons of different sizes.

In some embodiments, the at least one dissection balloon includes a plurality of oval balloons of different sizes.

In certain embodiments, the at least one dissection balloon includes at least one round balloon and at least one oval balloon.

In embodiments, the at least one spacer has a shape of a disc having a lateral slit therein and a hole at its center.

An exemplary method of forming a balloon dissector assembly for use in a system for dissecting tissue is disclosed including the steps of obtaining a kit including a balloon dissector assembly having an elongate shaft and a distal tip, and a plurality of dissection balloons, wherein each of the dissection balloons has an inflatable portion and a connector for releasable attachment to the distal tip; selecting one of the plurality of dissection balloons for use; and releasably attaching the selected dissection balloon to the distal tip of the elongate shaft.

In embodiments, the kit further includes at least one spacer for limiting a depth of insertion of the elongate shaft of the balloon dissector assembly into a body of a patient.

The exemplary method further includes placing the at least one spacer along the elongate shaft of the balloon dissector assembly to limit a depth of insertion of the elongate shaft of the balloon dissector assembly into a body of a patient.

The exemplary method further includes placing a seal between the distal tip of the elongate shaft and the connector.

In embodiments, the distal tip of the elongate shaft and the connector are threadably attached.

In embodiments, the distal tip of the elongate shaft is releasably attached to the connector by a bayonet configuration.

In embodiments, the distal tip of the elongate shaft is releasably attached to the connector by a locking collar.

In embodiments, a round balloon of desired size is selected as the dissection balloon.

In embodiments, an oval balloon of desired size is selected as the dissection balloon.

In embodiments, the at least one spacer has a shape of a disc having a lateral slit therein and a hole at its center. The lateral slit is placed along the elongate shaft of the dissector assembly and the spacer is pushed perpendicular to the axis of the elongate shaft so that the sides of the slit move away from the shaft until the shaft lies within the hole in the center of the spacer.

### BRIEF DESCRIPTION OF THE DRAWINGS

Various embodiments are described herein with reference to the drawings wherein:
FIG. 1 is a perspective view of a balloon dissector capable of attachment to multiple balloons in accordance with an embodiment of the present disclosure;
FIG. 2 is a perspective view of the balloon dissector shown in FIG. 1 having a single inflated balloon attached thereto in accordance with the present disclosure;
FIG. 3 includes cross-sectional views of various round balloons that may be provided with a kit in accordance with the present disclosure;
FIG. 4 includes cross-sectional views of various oval balloons that may be provided with a kit in accordance with the present disclosure;
FIG. 5 is a cross-sectional view of the distal tip of the balloon dissector shown in FIG. 1 threadably attached to a balloon in accordance with the present disclosure;
FIG. 6 is an enlarged cross-sectional view of the indicated area of detail shown in FIG. 5;
FIG. 7 is a cross-sectional view showing details of the threads for the balloon dissector and balloon shown in FIG. 6 in accordance with the present disclosure;
FIG. 8 is a perspective view of the balloon dissector and balloon for an alternate embodiment of the present disclosure, where the balloon dissector and balloon are connected by a bayonet attachment method;
FIG. 9 is an enlarged view of the coupling structure of the balloon dissector and balloon in accordance with the embodiment of FIG. 8;
FIG. 10 is an enlarged view of the coupling structure of the balloon dissector and balloon for assembly in accordance with the embodiment of FIG. 8;
FIG. 11 is an enlarged view showing steps for attaching the balloon dissector and balloon in accordance with the embodiment of FIG. 8;
FIG. 12 is an enlarged view showing the balloon dissector attached to the balloon after assembly in accordance with the embodiment of FIGS. 8-11;
FIG. 13 is a perspective view of the balloon dissector and balloon attached using a locking collar in accordance with the present disclosure;
FIG. 14 is an enlarged view of the assembly of the balloon dissector and balloon in accordance with the embodiment of FIG. 13;
FIG. 15 is an enlarged view showing attachment of the balloon dissector and balloon in accordance with the embodiment of FIGS. 13-14;
FIG. 16 is an enlarged view showing attachment of the balloon dissector and balloon in accordance with the embodiment of FIGS. 13-16;
FIG. 17 is a perspective view of a spacer to be used with a kit of the present disclosure;
FIG. 18 is a perspective view of a variable length system formed of a kit of the present disclosure;
FIG. 19 is a perspective view of a variable length system formed of a kit of the present disclosure using spacers to shorten the working length of the system;
FIG. 20 is a perspective view of a variable length system of the present disclosure without spacers; and
FIG. 21 is an exploded view of the components of a variable length system formed of a kit of the present disclosure.

### DETAILED DESCRIPTION

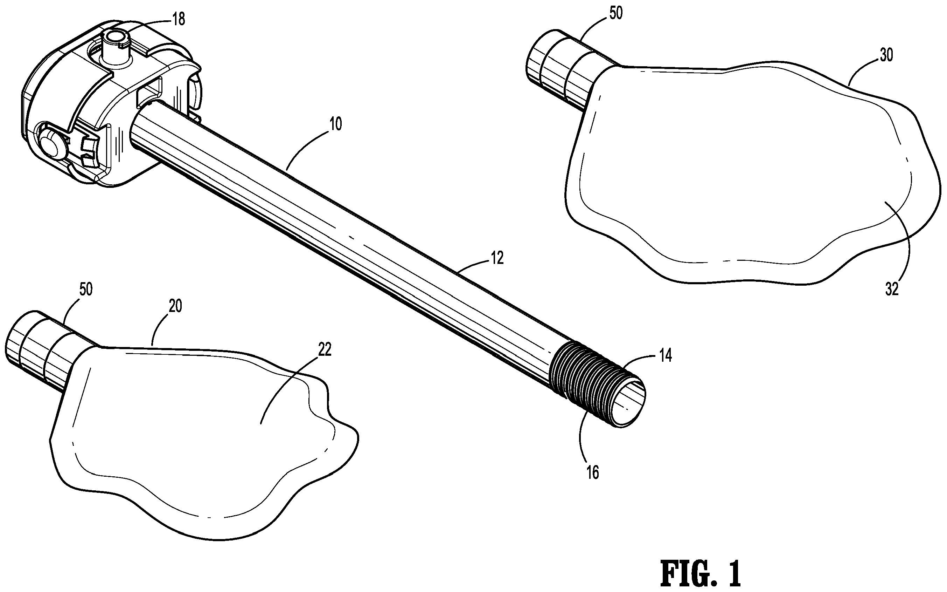

Dissection and access kits including a balloon dissector assembly in accordance with embodiments of the present disclosure are shown in FIGS. 1-21. The kits include a balloon dissector assembly including an elongated shaft and a plurality of detachable balloons connectable to the elongate shaft. The provision of detachable balloons allows a surgeon to select a balloon having a configuration best suited for a particular surgical procedure as discussed in detail below.

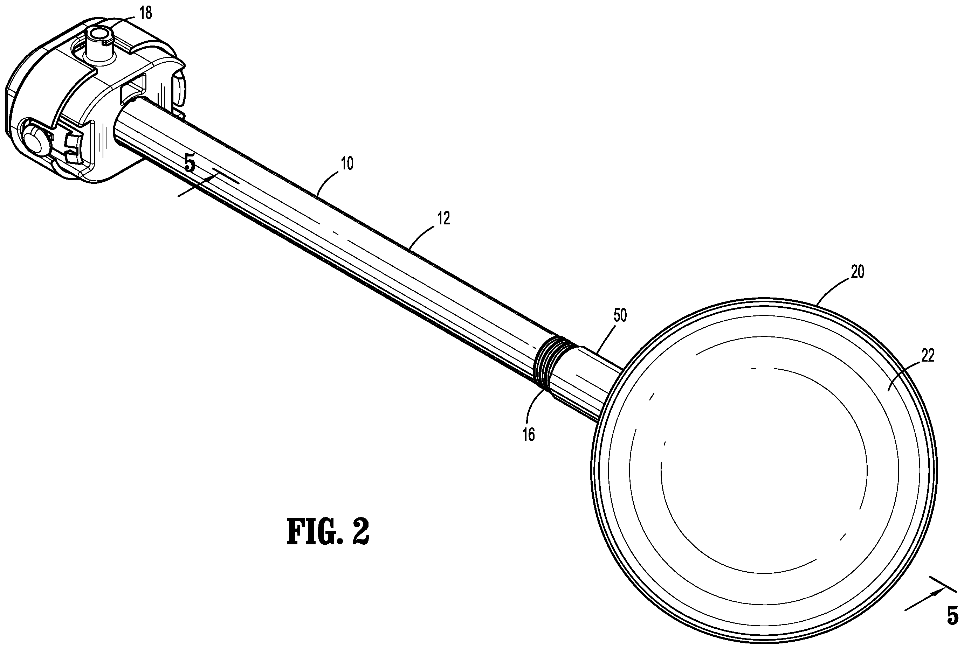

Referring to FIGS. 1 and 2, the kit of the present disclosure includes a balloon dissector assembly 10 having an elongate shaft 12 and a distal tip 14, with varying balloons 20 and 30, having different shapes and sizes, for attachment to distal tip 14 of the elongate shaft 12. Balloons 20 and 30 include both an inflatable portion 22, 32, and a connector 50, 50a, for attachment with the distal tip 14 of the elongate shaft 12 of the balloon dissector assembly 10. As depicted in FIG. 1, in embodiments the distal tip 14 may have threads 16 suitable for engaging threads (not shown) within the connector 50, 50a by threadably attaching together the distal tip 14 and the connector 50, 50a.

The elongate shaft 12 includes an inflation port 18 for inflating the dissection balloon 20, 30. In order to inflate the dissection balloon 20, 30 a source of inflation pressure is releasably attached to the inflation port 18 and pressurized fluid is introduced through the inflation port 18 and communicated along axis 5-5 to the dissection balloon 20, 30. FIG. 2 shows the balloon dissector assembly 10 with the balloon 20 attached thereto and inflated.

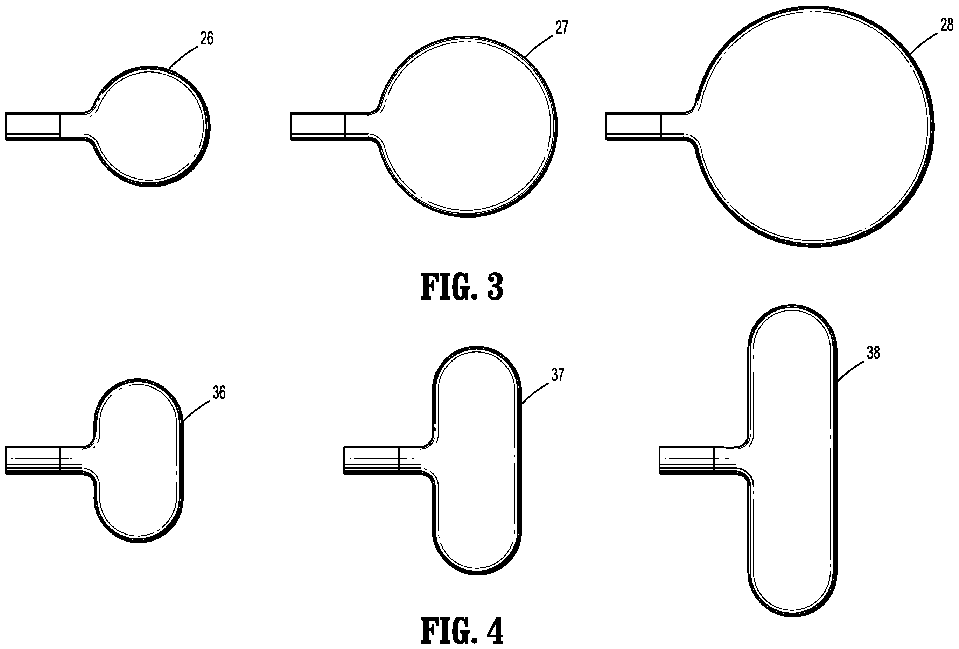

As noted above, balloons of varying shapes and sizes may be supplied with a kit of the present disclosure permitting increased variability in the procedures that require dissector balloons of varying shapes. Suitable shapes include round balloons as depicted in FIG. 3 as round balloons 26, 27, and 28, and oval balloons as depicted in FIG. 4 as oval balloons 36, 37, and 38. The balloons may be elastic, inelastic or a combination of materials having both characteristics. The selection of balloon is left up to the surgeon. In some cases, a round balloon may be made of an elastic material and a laterally extending oval balloon may be made of an inelastic material.

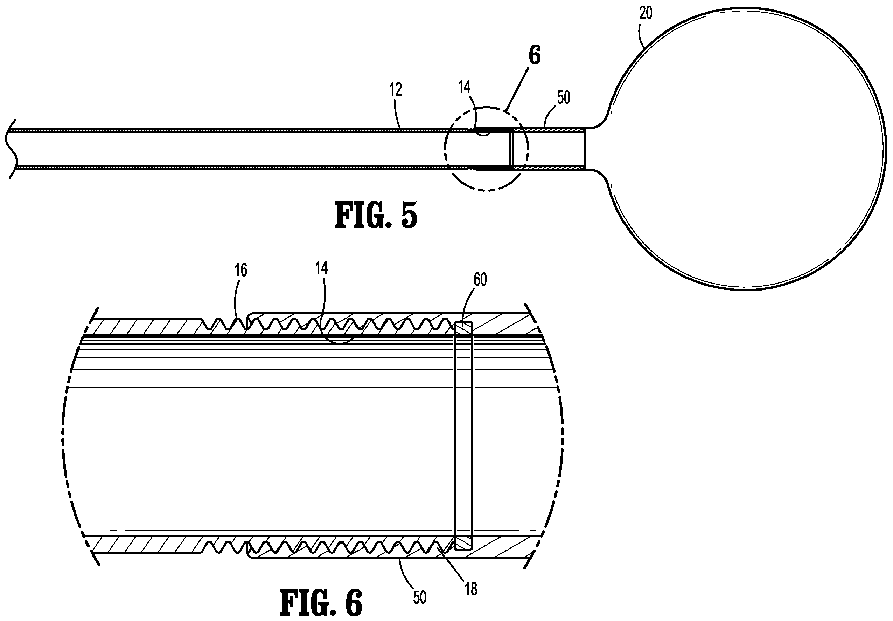

Turning to FIGS. 5-7, greater detail of the attachment of the connector 50 of the balloon 20 for attachment with the distal tip 14 of the elongate shaft 12 of the balloon dissector assembly 10 is shown. As depicted in FIGS. 5, 6 and 7, the distal tip 14 of the elongate shaft 12 is of a male configuration having a smaller circumference than the circumference of the connector 50, which has a female configuration. The distal tip 14 has threads 16 thereon suitable for engaging threads 18 within the connector 50 for threadably attaching the distal tip 14 and the connector 50. The connector 50 of the balloon 20 also possesses a seal 60 disposed therein, which permits attachment of the balloon 20 to the distal tip 14 in a secure and air-tight manner.

While FIGS. 5-7 depict the distal tip 14 of the elongate shaft 12 having a male configuration and the connector 50 having a female configuration, in embodiments (not shown) reverse configurations are contemplated, i.e., the distal tip 14 having a female configuration and the connector 50 having a male configuration.

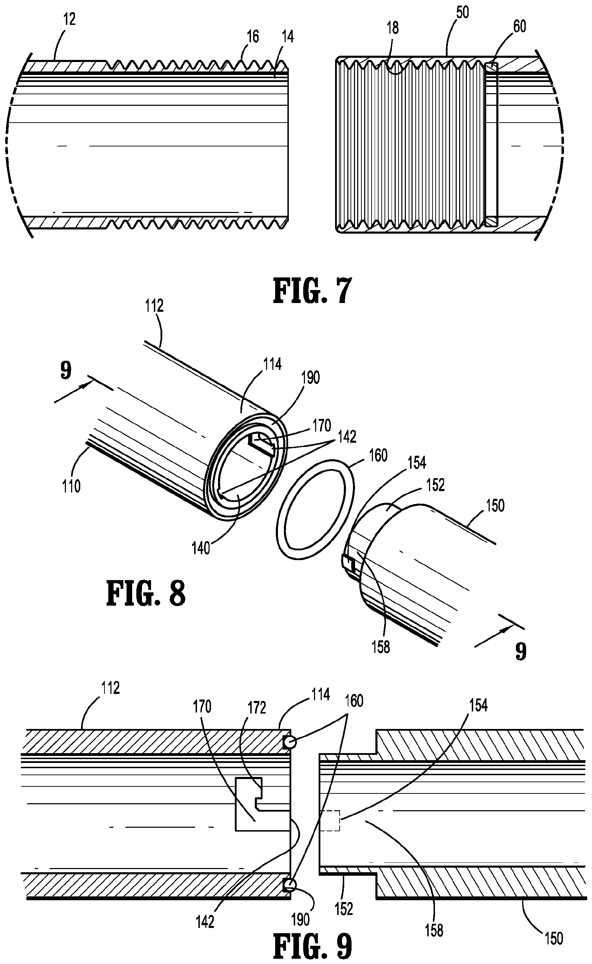

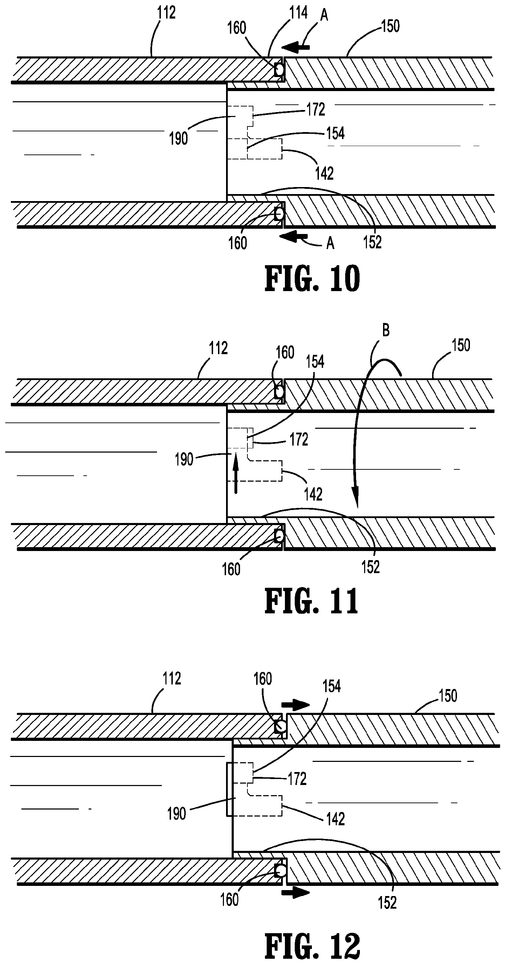

Other coupling configurations for attaching a distal tip 114 of an elongate shaft 112 of a balloon dissector assembly 110 with a connector 150 of balloons (not shown) are contemplated. For example, as depicted in FIGS. 8-12, a bayonet configuration for attaching the distal tip 114 with the connector 150 is provided. As depicted in FIG. 8, the distal tip 114 of the elongate shaft 112 of the balloon dissector assembly 110 possesses an open end 140 with slots 142 on the inner surface of the elongate shaft 112. As depicted in FIGS. 8 and 9, the slots 142 extend proximally along an inner wall of the elongate shaft 112, thereby forming grooves 170 thereon. (A second slot 142 forms a second corresponding groove, 170, not shown in FIGS. 8 or 9, but shown in FIG. 11.) The connector 150 of the balloon 120 possesses a proximal end 152 having a male configuration which is received within the open end 140, having a female configuration, of the elongate shaft 112. In addition, an outer surface 158 of the proximal end 152 of the connector 120 has tabs 154 (second tab 154 is not shown) extending from the outer surface 158 of the connector 120. The tabs 154 are slidably received within the slots 142 of the open end 140 of the elongate shaft 112. As shown in FIG. 8, the open end 140 of the elongate shaft 112 may also possess a recess 190 therein for placement of a seal 160, e.g., an o-ring, which may be placed between the elongate shaft 112 and the connector 120.

Turning to FIGS. 9-12, in use, the seal 160 is placed in the recess 190 in the open end 140 of the elongate shaft 112 of the balloon dissector assembly 110 (FIG. 9). The proximal end 152 of the connector 150 is then inserted into the open end 140 of the elongate shaft 112 in the direction indicated by arrows "A" in FIG. 10 so that the tabs 154 extending from the outer surface 158 of the connector 150 are slidably received in the slots 142 of the open end 140 of the elongate shaft 112. The connector 150 and the distal tip 114 of the elongate shaft 112 are then pressed together, with the seal 160 compressed and acting like a spring, so that the tabs 154 travel proximally from the slots 142 to the grooves 170 along axis 9-9. As shown in FIG. 11, as the grooves 170 possess an "L" shape, once the tabs 154 can travel no further in the proximal direction along the axis 9-9, the connector 120 is rotated around the axis 9-9 in the direction indicated by arrow "B" in FIG. 11 so that the tabs 154 travel along the grooves 170 in a direction perpendicular to the axis 9-9. As shown in FIG. 12, upon release of the connector 150 and the distal tip 114 of the elongate shaft 112, the seal 160, again acting like a spring, is decompressed, and permits the tabs 154 to move distally along the axis 9-9 into the recesses 172 (only 1 recess is depicted) at the end of the grooves 170 which lock the tabs 154 into the grooves 170, preventing further twisting or movement of the connector 120 relative to the elongate shaft 112 (in the absence of additional compressive/twisting forces).

While FIGS. 8-12 depict the distal tip 114 having certain structure and the connector 150 having corresponding structures to permit attachment of the distal tip 114 to the connector 150 by a bayonet attachment configuration, in embodiments (not shown) reverse configurations are contemplated, i.e., the distal tip 114 possesses the structures shown for the connector 150 and the connector 150 possesses the structures for the distal tip 114.

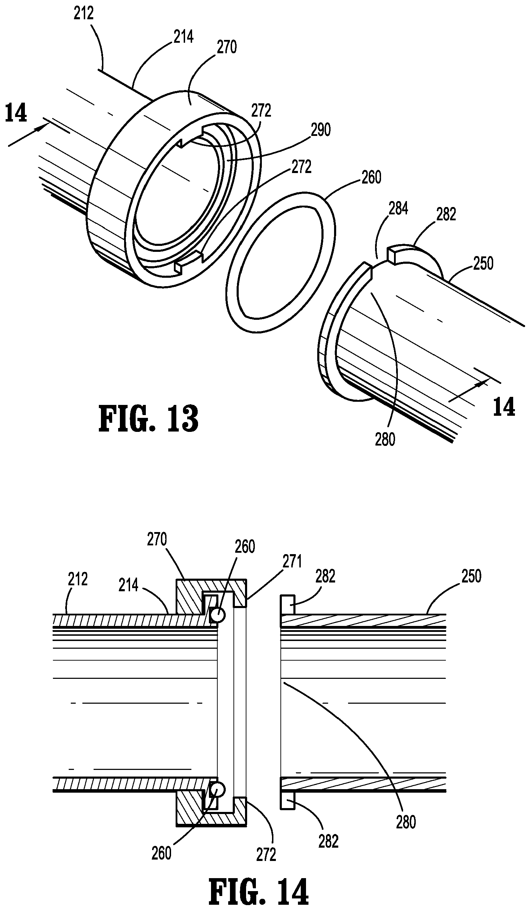

Yet another configuration for attaching a distal tip 214 of an elongate shaft 212 of a balloon dissector assembly with a connector 250 of a balloon is depicted in FIGS. 13-16, which show a locking collar configuration for attaching the distal tip 214 of the elongate shaft 212 with the connector 250. As depicted in FIG. 13, the distal tip 214 of the elongate shaft 212 of a balloon dissector assembly possesses a locking collar 270 thereon with protrusions 272 on the inner surface of the locking collar 270. As depicted in FIGS. 13 and 14, the connector 250 possesses a proximal end 280 with an incomplete ring 282 thereon having gaps 284, (second gap 284 is not shown in FIG. 13), which are configured to allow the protrusions 272 of the locking collar 270 to pass distally beyond the ring 282. As shown in FIG. 13, the elongate shaft 212 may also possess a recess 290 therein for placement of a seal 260, which may be placed between the elongate shaft 212 and the connector 250 of the balloon.

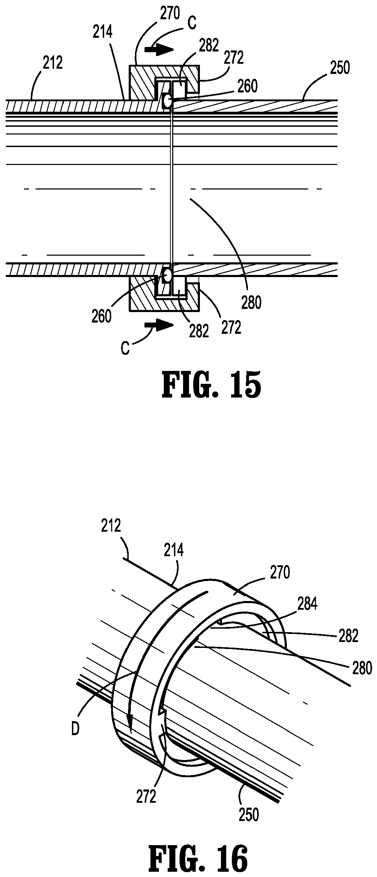

Turning to FIGS. 15 and 16, in use, the seal 260 is placed in the recess 290 of the distal tip 214 of the elongate shaft 212 of the balloon dissector assembly. The locking collar 270 is then placed over the proximal end 280 of the balloon connector 250 in the direction indicated by arrow "C" in FIG. 15 so that the protrusions 272 on the inner surface of the locking collar 270 can pass through the gaps 284 of the incomplete ring 282. The connector 250 and the distal tip 214 of the elongate shaft 212 are then pressed together, with the seal 260 compressed and acting like a spring, so that the protrusions 272 pass through the gaps 284, allowing the distal end of the locking collar 270 to pass distally beyond the ring 282. The locking collar 270 is then rotated around axis 14-14 in a direction indicated by arrow "D" in FIG. 16, so that the protrusions 272 on the inner surface of the locking collar 270 abut the incomplete ring 282. Upon release of the locking collar 270, the seal 260, again acting like a spring, is decompressed, and the protrusions 272 frictionally engage with the incomplete ring 282, preventing further twisting or movement of the locking collar 270 relative to the balloon connector 250 (in the absence of additional compressive/twisting forces).

While FIGS. 13-16 depict the distal tip 214 of the elongate shaft 212 having certain structure and the connector 250 having corresponding structures to permit attachment of the distal tip 214 to the connector 250 by the locking collar 270, in embodiments (not shown) reverse configurations are contemplated, i.e., the distal tip 214 possesses the structures shown for the connector 250 and the connector 250 possesses the structures for the distal tip 214.

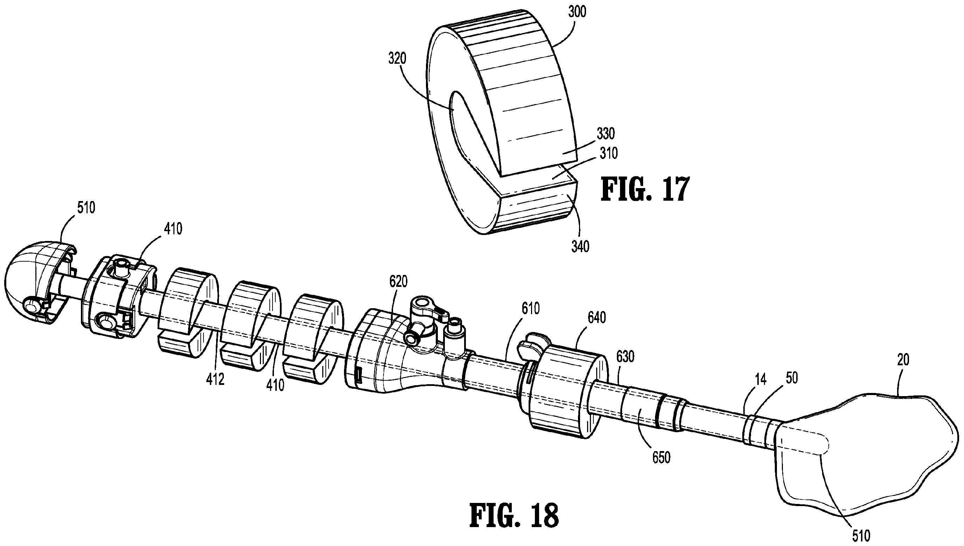

Referring to FIGS. 17 and 18, the balloon dissector assembly 410 of the present disclosure may be utilized with an obturator 510 and a cannula assembly 610 for use in endoscopic and laparoscopic minimally invasive procedures. An access cannula 630 of the cannula assembly 610 remains in place for use during the minimally invasive procedure, and the obturator 510 includes a sharp tip for penetrating the body cavity.

In embodiments, to further enhance the variability of the kit of the present disclosure, removable spacers 300 may be provided to vary the working length of the balloon dissector assembly 410. More specifically, the removable spacers 300 may be added between the dissector assembly 410 and the cannula assembly 610 to reduce the overall working length of the shaft 12 of the balloon dissector assembly 410, i.e., to reduce the extent to which the balloon dissector assembly 10 extends from the distal end of cannula assembly 610 into a body cavity. Suitable spacers 300 are depicted in FIG. 17. Each spacer 300 is in the shape of a disc, having a lateral slit 310 therein and a hole 320 in the center of the spacer 300. The slit 310 permits sliding of the spacer 300 onto the elongate shaft 412 of the balloon dissector assembly 410 in such a way that when the slit 310 is placed adjacent the shaft 412 and the spacer 300 is pushed perpendicular to the axis of the elongate shaft 412, the sides 330, 340 of the slit 310 are moved away from the shaft 412 until the shaft 412 comes to lie within the hole 320 in the center of the spacer 300, at which point the sides 330, 340 of the slit 310 return to their original configuration and the spacer 300 encompasses the shaft 412.

The provision of a spacer between the cannula assembly 610 and the balloon dissector assembly 410 makes it possible to vary the depth of insertion of a balloon 20 of the balloon dissector assembly 410 of the present disclosure into a body cavity. Placement of more than one spacer 300 may further limit the depth of insertion of the balloon dissector assembly 410, thus providing a practitioner with a means to vary depth of insertion to suit the needs of a patient in view of the procedure being performed. As the spacer(s) 300 are releasably mounted on the instrument, the spacer 300 can be fitted on the medical instrument when the need arises. Moreover, the releasability of the spacer 300 permits altering the depths of insertion of the balloons 20, 30 of the balloon dissector assembly 410. This can be done so that the use of the balloon dissector assembly 410 can be adapted with the greatest possible variation to the anatomical circumstances in question.

FIG. 18 depicts a system of the present disclosure showing the balloon dissector assembly 410, the dissector obturator 510, and the balloon cannula assembly 610. Spacers 300, 320, and 330 are shown as they would be placed on the outer surface of the elongate shaft 412 of the balloon dissector assembly 410, to adjust the working length of the system. The cannula assembly 610 includes a cannula housing 620 and the access cannula 630 with a skin seal 640 and an anchoring balloon 650 thereon. The dissector obturator assembly 510 and the balloon dissector assembly 410 are insertable into the access cannula 630.

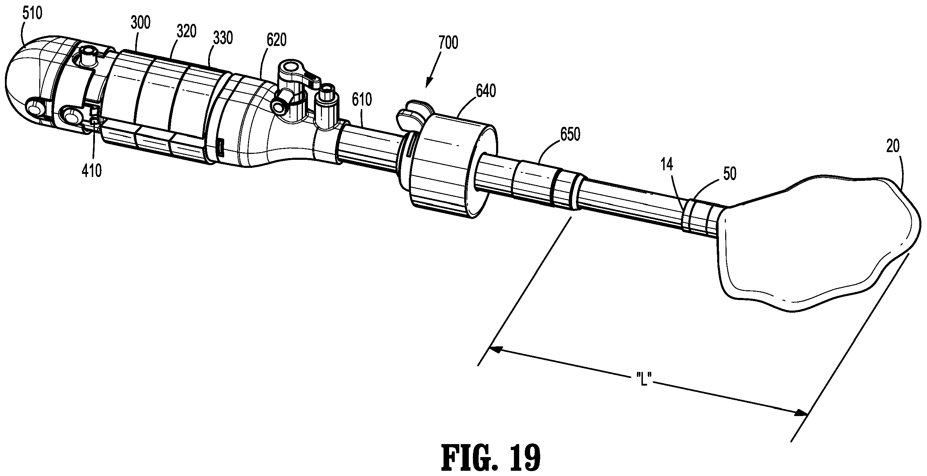

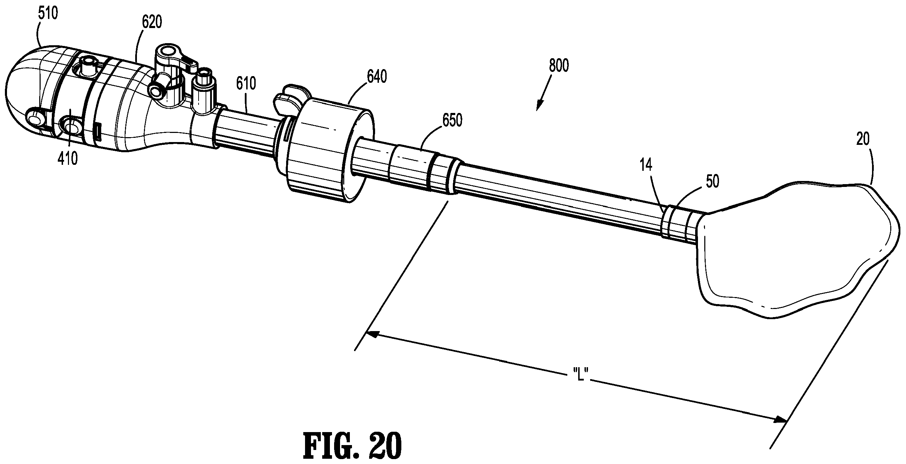

FIG. 19 is a depiction of a variable length system 700, showing placement of the spacers 300, 320, and 330 on the outer surface of the elongate shaft 412 of the balloon dissector assembly 410, giving a defined working length "L" for the system. FIG. 20 shows a similar variable length system 800, lacking any spacers on the outer surface of the elongate balloon dissector assembly 410, giving a larger defined working length "L'" for the system compared with FIG. 19.

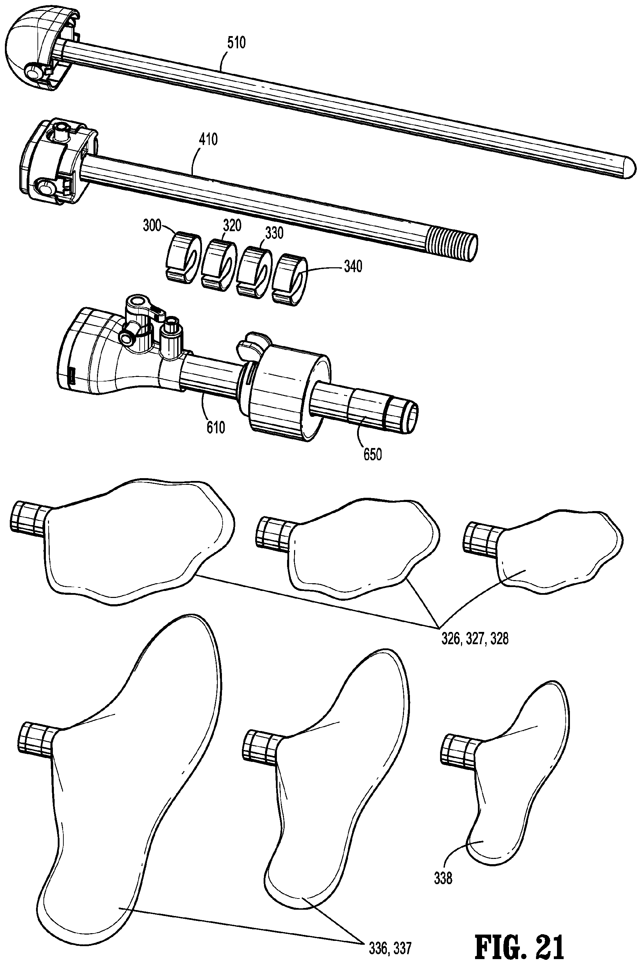

FIG. 21 is a depiction of the unassembled variable length system of the present disclosure, showing the balloon dissector assembly 410, separate from the dissector obturator 510, and the balloon cannula assembly 610. The spacers 300, 320, 330, and 340 are shown separately, as are the round balloons 326, 327, and 328, and the oval balloons 336, 337, and 338.

Additional details regarding the system, including the components utilized in the system, methods for its production, and methods for its use, include those disclosed in U.S. Patent No. 8,540,745.

The balloon dissector assembly 410 is used for dissecting tissue along natural tissue planes in general, laparoscopic, vascular endoscopic, plastic or reconstructive surgery or other procedures requiring the separation of tissue. Where prior devices would require different working lengths and different balloons thereon, requiring multiple systems on hand for use in a procedure, kits of the present disclosure permit customization of both the balloon utilized for dissection as well as the working length of the system.

The material of the balloon may be elastic, so as to follow a path of least resistance in the body, inelastic so as to assume a predetermined shape upon inflation, or a combination of elastic and inelastic materials. The balloon dissector and cannula assembly may be used in hernia repair, bladder neck suspension or other procedures requiring the separation of tissue. The balloon dissector and cannula assemblies can be made from any medical grade material, including metals and plastics. The apparatus is made using well-known techniques.

In embodiments, round balloons may be formed of an elastic material, while laterally extending oval balloons may be inelastic.

The dissection balloon may be inflated with any medical grade fluid, such as saline, CO₂, or any other fluid. The balloons may be inflated using a syringe, mechanically or manually operated pump or other means. The ports for inflating the balloons may be used with one-way valves, check valves, or any other valve arrangement for inflating the balloons.

The shape of the dissection balloon can vary upon the area of use in the anatomical structure. For example, the balloon may have the round shape of a globe, a flattened round shape, may include a longitudinally oval shape or other shapes such as kidney shaped, laterally extending, etc., depending on the need of the surgeon. The selection of balloon is left up to the surgeon. Once the desired balloon is selected by the surgeon, it is affixed to the distal tip of the elongate shaft of the balloon dissector assembly as described in the above embodiments, which include by use of threadable attachment, the use of a bayonet attachment, the use of a locking collar, etc.

Similarly, as noted above, the desired working length of the system may be optimized for the patient in view of the intended procedure with the use of the spacers affixed to the elongate shaft of the balloon dissector assembly, and/or any other shaft of the dissector obturator or balloon cannula assembly. Again, the more spacers added to the shaft, the shorter the working length of the system, which is decided upon by the surgeon at the time of the procedure.

Once the kit of the present disclosure has been used to assemble a desired system, a suitably sized incision is made in the patient's skin. Next, the assembled balloon dissector assembly 410 and the balloon cannula assembly 610 are inserted into the incision, using the dissector obturator 510 to tunnel a passage beyond the point of incision.

Inflation pressure is supplied through the inflation port 28 from a suitable outside source and is communicated to the dissection balloon 20. As pressure is applied, the dissection balloon 20 expands. The expansion of dissection balloon dissects surrounding tissue along natural tissue planes. Once the desired space is created, the dissection balloon 20 is deflated by disengaging the inflation source from the inflation port 28 and/or connecting the inflation port 20 to a source of vacuum.

The balloon dissector assembly 410 is removed from the cannula assembly 610 and surgical instruments are introduced to the surgical site through the cannula. Examples of such surgical instruments include, but are not limited to, endoscopes, surgical suturing devices, and surgical device applicators.

For example, in alternative embodiments, the dissector obturator 510 is removed and replaced with an endoscope. Then, the balloon dissector assembly 410 and the cannula assembly 610 are inserted into the skin incision and the dissector balloon 20 is inflated as discussed above. The scope is used for supporting the balloon 20, as well as for viewing the space to be dissected prior to, during, and after dissection.

It will be understood that various modifications may be made to the embodiments disclosed herein. For example, other configurations of securing a balloon to a balloon dissector assembly may be provided to form a system of the present disclosure. Additionally, other balloon shapes and construction such as, for example elastic, inelastic, oval, kidney shaped, along with constructions providing differential expansion characteristics may be provided. Further, the terminology of similar components with the various embodiments should not be construed as specific to any particular embodiment. Thus, the above description should not be construed as limiting, but merely as exemplifications of preferred embodiments. Those skilled in the art will envision other modifications within the scope of the claims appended hereto.

## Claims

1. A kit for a dissection and access system comprising:
a cannula assembly (610) having a cannula housing (620) and an access cannula (630);
an obturator (510) insertable into the access cannula;
a dissector assembly (10; 110; 410) insertable into the access cannula, the dissector assembly having an elongate shaft (12; 112; 212; 412) having a distal tip (14; 114; 214); and
at least one dissection balloon (20, 30) including an inflatable portion (22, 32) and a connector (50; 50a; 150; 120; 250) for releasable attachment to the distal tip of the elongate shaft; and
a compressible seal (60; 160; 260) for placement between the distal tip of the elongate shaft and the connector.

2. The kit of claim 1, further comprising at least one spacer (300) releasably attachable to the elongate shaft (12; 112; 212; 412) for limiting a depth of insertion (L) of the elongate shaft of the dissector assembly (10; 110; 410) into a body of a patient; and/or wherein the distal tip (14) of the elongate shaft and the connector (50) possess threads (16) permitting the distal tip and connector to be threadably attached.

3. The kit of any preceding claim, wherein the distal tip (114) of the elongate shaft (112) and the connector (150) include structure permitting the distal tip to be releasably attachable to the connector by a bayonet configuration; preferably wherein the distal tip possesses an open end with slots (142) on an inner surface of the elongate shaft, the slots extending proximally along the inner wall of the elongate shaft to form recesses thereon, the connector having a proximal end configured to fit within the open end of elongate shaft, an outer surface of the proximal end having tabs (154) extending from the outer surface of the connector, the tabs being configured to be slidably received within the slots of the open end of the elongate shaft.

4. The kit of any preceding claim, further comprising a locking collar (270) for releasably attaching the distal tip (214) of the elongate shaft (212) to the connector (250).

5. The kit of any preceding claim, wherein the at least one dissection balloon (20, 30) includes a plurality of round balloons of different sizes and/or wherein the at least one dissection balloon includes a plurality of oval balloons of different sizes.

6. The kit of claim 5, wherein the at least one dissection balloon (20, 30) includes at least one round balloon and at least one oval balloon.

7. The kit of claim 1, wherein the at least one spacer (300) has a shape of a disc, the disc having a lateral slit (310) therein and a hole (320) at its center.

8. A method of forming a balloon dissector assembly (10; 110; 410) for use in a system for dissecting tissue comprising:
obtaining a kit including a balloon dissector assembly (10; 110; 410) having an elongate shaft (12; 112; 212; 412) and a distal tip (14; 114; 214), a plurality of dissection balloons (20, 30), each of the dissection balloons having an inflatable portion (22, 32) and a connector (50; 50a; 150; 120; 250) for releasable attachment to the distal tip;
selecting one of the plurality of dissection balloons for use;
placing a compressible seal (60; 160; 260) between the distal tip of the elongate shaft and the connector; and
releasably attaching the selected dissection balloon to the distal tip of the elongate shaft.

9. The method of claim 8, wherein the kit further includes at least one spacer (300) for limiting a depth of insertion of the elongate shaft (12; 112; 212; 412) of the balloon dissector assembly (10; 110; 410) into a body of a patient and the method further includes placing the at least one spacer along the elongate shaft of the balloon dissector assembly to limit a depth of insertion (L) of the elongate shaft of the balloon dissector assembly into a body of a patient.

10. The method of claim 8 or claim 9, wherein the distal tip (14) of the elongate shaft (12) is releasably attached to the connector by threads (16); and/or wherein the distal tip (114) of the elongate shaft (112) is releasably attached to the connector (150) by a bayonet configuration; and/or wherein the distal tip (214) of the elongate shaft (212) is releasably attached to the connector (250) by a locking collar (270).

11. The method of any of claims 8 to 10, wherein a round balloon of desired size is selected as the dissection balloon (20, 30); or wherein an oval balloon of desired size is selected as the dissection balloon (20, 30).

12. The method of any of claims 8 to 11, wherein the at least one spacer (300) has a shape of a disc, the disc having a lateral slit (310) therein and a hole (320) at its center, and the method further includes placing the lateral slit along the elongate shaft (12; 112; 212; 412) of the dissector assembly (10; 110; 410) and pushing the spacer perpendicular to the axis of the elongate shaft so that the sides (330, 340) of the slit move away from the shaft until the shaft lies within the hole in the center of the spacer.

## Patentansprüche

1. Kit für ein Dissektions- und Zugangssystem umfassend:
eine Kanülenanordnung (610) mit einem Kanülengehäuse (620) und einer Zugangskanüle (630);
einen Obturator (510), der in die Zugangskanüle einführbar ist;
eine Dissektoranordnung (10; 110; 410), die in die Zugangskanüle einführbar ist, wobei die Dissektoranordnung einen länglichen Schaft (12; 112; 212; 412) mit einer distalen Spitze (14; 114; 214) aufweist; und
zumindest einen Dissektionsballon (20, 30), der einen aufblasbaren Abschnitt (22, 32) und einen Verbinder (50; 50a; 150; 120; 250) zur lösbaren Befestigung an der distalen Spitze des länglichen Schafts aufweist; und
eine komprimierbare Dichtung (60; 160; 260) zur Platzierung zwischen der distalen Spitze des länglichen Schafts und dem Verbinder.

2. Kit nach Anspruch 1, weiter umfassend zumindest einen Abstandhalter (300), der an dem länglichen Schaft (12; 112; 212; 412) lösbar befestigt werden kann, um eine Einführungstiefe (L) des länglichen Schafts der Dissektoranordnung (10; 110; 410) in einen Körper eines Patienten zu begrenzen; und/oder
wobei die distale Spitze (14) des länglichen Schafts und der Verbinder (50) Gewinde (16) besitzen, die eine schraubbare Befestigung der distalen Spitze und des Verbinders erlauben.

3. Kit nach einem vorstehenden Anspruch, wobei die distale Spitze (114) des länglichen Schafts (112) und der Verbinder (150) Struktur aufweisen, die es erlaubt, dass die distale Spitze durch eine Bajonett-Konfiguration lösbar an dem Verbinder befestigt werden kann; wobei die distale Spitze bevorzugt ein offenes Ende mit Schlitzen (142) an einer Innenfläche des länglichen Schafts besitzt, wobei sich die Schlitze proximal entlang der Innenwand des länglichen Schafts erstrecken, um Aussparungen daran zu bilden, wobei der Verbinder ein proximales Ende aufweist, das konfiguriert ist, um in das offene Ende des länglichen Schafts zu passen, wobei eine Außenfläche des proximalen Endes Laschen (154) aufweist, die sich von der Außenfläche des Verbinders erstrecken, wobei die Laschen konfiguriert sind, um in den Schlitzen des offenen Endes des länglichen Schafts gleitend empfangen zu werden.

4. Kit nach einem vorstehenden Anspruch, weiter umfassend einen Verriegelungskragen (270) zum lösbaren Befestigen der distalen Spitze (214) des länglichen Schafts (212) an dem Verbinder (250).

5. Kit nach einem vorstehenden Anspruch, wobei der zumindest eine Dissektionsballon (20, 30) eine Vielzahl von runden Ballons unterschiedlicher Größen aufweist und/oder wobei der zumindest eine Dissektionsballon eine Vielzahl von ovalen Ballons unterschiedlicher Größen aufweist.

6. Kit nach Anspruch 5, wobei der zumindest eine Dissektionsballon (20, 30) zumindest einen runden Ballon und zumindest einen ovalen Ballon aufweist.

7. Kit nach Anspruch 1, wobei der zumindest eine Abstandhalter (300) die Form einer Scheibe hat, wobei die Scheibe einen seitlichen Schlitz (310) darin und ein Loch (320) in ihrer Mitte aufweist.

8. Verfahren zur Bildung einer Ballondissektoranordnung (10; 110; 410) zur Verwendung in einem Gewebedissektionssystem umfassend:
Erhalten eines Kits, das eine Ballondissektoranordnung (10; 110; 410) mit einem länglichen Schaft (12; 112; 212; 412) und einer distalen Spitze (14; 114; 214), eine Vielzahl von Dissektionsballons (20, 30) aufweist, wobei jeder der Dissektionsballons einen aufblasbaren Abschnitt (22, 32) und einen Verbinder (50; 50a; 150; 120; 250) zur lösbaren Befestigung an der distalen Spitze aufweist;
Auswählen eines der Vielzahl von Dissektionsballons zur Verwendung;
Platzieren einer komprimierbaren Dichtung (60; 160; 260) zwischen der distalen Spitze des länglichen Schafts und dem Verbinder; und
lösbar Befestigen des ausgewählten Dissektionsballons an der distalen Spitze des länglichen Schafts.

9. Verfahren nach Anspruch 8, wobei das Kit weiter zumindest einen Abstandhalter (300) zum Begrenzen einer Einführungstiefe des länglichen Schafts (12; 112; 212; 412) der Ballondissektoranordnung (10; 110; 410) in einen Körper eines Patienten aufweist und das Verfahren weiter Platzieren des zumindest einen Abstandhalters entlang des länglichen Schafts der Ballondissektoranordnung, um eine Einführungstiefe (L) des länglichen Schafts der Ballondissektoranordnung in einen Körper eines Patienten zu begrenzen, aufweist.

10. Verfahren nach Anspruch 8 oder Anspruch 9, wobei die distale Spitze (14) des länglichen Schafts (12) durch Gewinde (16) an dem Verbinder lösbar befestigt ist; und/oder wobei die distale Spitze (114) des länglichen Schafts (112) durch eine Bajonett-Konfiguration an dem Verbinder (150) lösbar befestigt ist; und/oder wobei die distale Spitze (214) des länglichen Schafts (212) durch einen Verriegelungskragen (270) an dem Verbinder (250) lösbar befestigt ist.

11. Verfahren nach einem der Ansprüche 8 bis 10, wobei ein runder Ballon einer gewünschten Größe als Dissektionsballon (20, 30) ausgewählt wird; oder wobei ein ovaler Ballon einer gewünschten Größe als Dissektionsballon (20, 30) ausgewählt wird.

12. Verfahren nach einem der Ansprüche 8 bis 11, wobei der zumindest eine Abstandhalter (300) die Form einer Scheibe hat, wobei die Scheibe einen seitlichen Schlitz (310) darin und ein Loch (320) in ihrer Mitte aufweist, und das Verfahren weiter aufweist Platzieren des seitlichen Schlitzes entlang des länglichen Schafts (12; 112; 212; 412) der Dissektoranordnung (10; 110; 410) und Schieben des Abstandhalters senkrecht zu der Achse des länglichen Schafts, so dass sich die Seiten (330, 340) des Schlitzes von dem Schaft weg bewegen, bis der Schaft in dem Loch in der Mitte des Abstandhalters liegt.

## Revendications

1. Kit pour système de dissection et d'accès comprenant :
un ensemble canule (610) ayant un boîtier de canule (620) et une canule d'accès (630) ;
un obturateur (510) pouvant être inséré dans la canule d'accès ;
un ensemble dissecteur (10; 110; 410) pouvant être inséré dans la canule d'accès, l'ensemble dissecteur ayant une tige allongée (12 ; 112 ; 212 ; 412) ayant une pointe distale (14 ; 114 ; 214) ; et
au moins un ballonnet de dissection (20, 30) incluant une partie gonflable (22, 32) et un raccord (50 ; 50a ; 150 ; 120 ; 250) pour la fixation libérable à la pointe distale de la tige allongée ; et
un joint compressible (60 ; 160 ; 260) pour le placement entre la pointe distale de la tige allongée et le raccord.

2. Kit selon la revendication 1, comprenant en outre au moins un écarteur (300) fixé de manière libérable à la tige allongée (12 ; 112 ; 212 ; 412) pour limiter une profondeur d'insertion (L) de la tige allongée de l'ensemble dissecteur (10 ; 110 ; 410) dans un corps d'un patient ; et/ou dans lequel la pointe distale (14) de la tige allongée et le raccord (50) possèdent des filetages (16) permettant que la pointe distale et le raccord soient fixés par filetage.

3. Kit selon l'une quelconque des revendications précédentes, dans lequel la pointe distale (114) de la tige allongée (112) et le raccord (150) incluent une structure permettant que la pointe distale soit fixée de manière libérable au raccord par une configuration de baïonnette ; de préférence dans lequel la pointe distale possède une extrémité ouverte avec des fentes (142) sur une surface intérieure de la tige allongée, les fentes s'étendant proximalement le long de la paroi intérieure de la tige allongée pour former des renfoncements sur celle-ci, le raccord ayant une extrémité proximale configurée pour s'ajuster à l'intérieur de l'extrémité ouverte de la tige allongée, une surface extérieure de l'extrémité proximale ayant des languettes (154) s'étendant à partir de la surface extérieure du raccord, les languettes étant configurées pour être reçues de façon coulissante à l'intérieur des fentes de l'extrémité ouverte de la tige allongée.

4. Kit selon l'une quelconque des revendications précédentes, comprenant en outre un collier de fixation (270) pour fixer de manière libérable la pointe distale (214) de la tige allongée (212) au raccord (250).

5. Kit selon l'une quelconque des revendications précédentes, dans lequel l'au moins un ballonnet de dissection (20, 30) inclut une pluralité de ballonnets ronds de différentes tailles et/ou dans lequel l'au moins un ballonnet de dissection inclut une pluralité de ballonnets ovales de différentes tailles.

6. Kit selon la revendication 5, dans lequel l'au moins un ballonnet de dissection (20, 30) inclut au moins un ballonnet rond et au moins un ballonnet ovale.

7. Kit selon la revendication 1, dans lequel l'au moins un écarteur (300) a une forme d'un disque, le disque ayant une fente latérale (310) à l'intérieur de celui-ci et un trou (320) en son centre.

8. Procédé de formation d'un ensemble dissecteur à ballonnet (10 ; 110 ; 410) pour son utilisation dans un système de dissection de tissu comprenant :
l'obtention d'un kit incluant un ensemble dissecteur à ballonnet (10; 110; 410) ayant une tige allongée (12 ; 112 ; 212 ; 412) et une pointe distale (14 ; 114 ; 214), une pluralité de ballonnets de dissection (20, 30), chacun des ballonnets de dissection ayant une partie gonflable (22, 32) et un raccord (50; 50a ; 150; 120; 250) pour la fixation libérable à la pointe distale ;
la sélection de l'un de la pluralité de ballonnets de dissection pour son utilisation ;
le positionnement d'un joint compressible (60; 160; 260) entre la pointe distale de la tige allongée et le raccord ; et
la fixation libérable du ballonnet de dissection sélectionné à la pointe distale de la tige allongée.

9. Procédé selon la revendication 8, dans lequel le kit inclut en outre au moins un écarteur (300) pour limiter une profondeur d'insertion de la tige allongée (12 ; 112 ; 212; 412) de l'ensemble dissecteur à ballonnet (10; 110; 410) dans un corps d'un patient et le procédé inclut en outre le positionnement de l'au moins un écarteur le long de la tige allongée de l'ensemble dissecteur à ballonnet pour limiter une profondeur d'insertion (L) de la tige allongée de l'ensemble dissecteur à ballonnet dans le corps d'un patient.

10. Procédé selon la revendication 8 ou la revendication 9, dans lequel la pointe distale (14) de la tige allongée (12) est fixée de manière libérable au raccord par les filetages (16) ; et/ou dans lequel la pointe distale (114) de la tige allongée (112) est fixée de manière libérable au raccord (150) par une configuration de baïonnette ; et/ou dans lequel la pointe distale (214) de la tige allongée (212) est fixée de manière libérable au raccord (250) par un collier de fixation (270).

11. Procédé selon l'une quelconque des revendications 8 à 10, dans lequel un ballonnet rond d'une taille souhaitée est sélectionné en tant que ballonnet de dissection (20, 30) ; ou dans lequel un ballonnet ovale d'une taille souhaitée est sélectionné en tant que ballonnet de dissection (20, 30).

12. Procédé selon l'une quelconque des revendications 8 à 11, dans lequel l'au moins un écarteur (300) a la forme d'un disque, le disque ayant une fente latérale (310) à l'intérieur de celui-ci et un trou (320) en son centre, et le procédé inclut en outre le positionnement de la fente latérale le long de la tige allongée (12 ; 112 ; 212 ; 412) de l'ensemble dissecteur (10; 110; 410) et le fait de pousser l'écarteur perpendiculaire à l'axe de la tige allongée de manière à ce que les côtés (330 ; 340) de la fente s'éloignent de la tige jusqu'à ce que la tige se trouve à l'intérieur du trou au centre de l'écarteur.
